# EUROPEAN PATENT APPLICATION

(11) **EP 2 258 322 A2**
(43) Date of publication of application: **08.12.2010**
(21) Application number: 10176874.5
(22) Date of filing: 02.09.2004
(51) Int. Cl.: A61F 2/44

(54) **Artificial intervertebral disc**

(30) Priority: 02.09.2003 US 653540
(62) Divisional of application: 04255310.7
(71) Applicant: Aesculap Implant Systems, Inc., Center Valley, PA 18034 (US)
(72) Inventor: Richelsoph, Marc, Bartlett, TN 38133 (US); Clift, Joseph S. Jr., Bartlett, TN 38133 (US)
(74) Representative: Jacob, Reuben Ellis

(57) **Abstract**

The invention relates to an artificial intervertebral disc comprising housing members including spaced inner surfaces facing each other and oppositely facing outer surfaces for engaging spaced apart intervertebral surfaces; and self adjusting bearing means operatively disposed between said inner surfaces for moving relative to said housing members to adjust and compensate for vertertebral disc motion, further including load sharing means disposed between said inner surfaces and about at least a portion of said bearing means for sharing absorption of compressive loads with said baring means while limiting the relative movement of said housing members.

## Description

The present invention relates generally to a spinal implant assembly for implantation into the intervertebral space between adjacent vertebral bones to provide stabilization and continued postoperative flexibility and proper anatomical motion. More specifically, the present invention relates to an artificial intervertebral disc, sometimes referred to as an intervertebral spacer device, for functioning as a load sharing and bearing device for replacement of the damaged, decayed, or otherwise nonfunctioning intervertebral disc.

The spine is a complex structure consisting of multiple flexible levels. Each level consists of a system of joints defined by adjacent vertebral bones. The system of joints includes intervertebral discs, which are a two-part structure. The disc consists of a nucleus and an annulus. The system allows motion while the facet joints add posterior stabilization to the spinal column. The disc allows motion and cushioning to the joint.

The complex system of the joint is subjected to varying loads and problems over time, including disc degeneration due to a variety of reasons. Disc degeneration can be attributed to aging, damage due to excessive loading, trauma, and other anatomical issues. Facet joints of the structure can be compromised due to the same reasons, as well as due to arthritic changes. Severe joint degeneration and failure can often cause sufficient pain to require surgical intervention.

The current standard method of treatment for severe pain caused by spine joint problems is fusion at the damaged level of the spine. The treatment, when successful, fuses the damaged section into a single mass of bone. The fusion of the joint eliminates motion of the joint, thereby reducing or eliminating pain at that level. Success rates for pain elimination are very high for this method of treatment. However, since the entire spine works as a system, fusion results in complications.

Elimination of motion at the spine alters the biomechanics of the spine at every other level. If one level is fused, then loads are absorbed by one less disc into a system not designed for such change. Thus, the remaining discs must redistribute loads, each disc absorbing a greater load. In addition, the spine flexes to absorb loads. A fusion alters the means by which the spine flexes, which also increases the loads on the remaining healthy discs. In turn, it is well understood that a complication of fusion is that additional fusions may be required in the future as the other discs deteriorate due to the altered biomechanics of the spine. In other words, short-term pain relief is exchanged for long-term alterations of the spine, which, in turn, usually require further surgery.

There are numerous prior art patents addressing the issue of disc replacement. The United States Patent Nos. 6,443,987 B1 and 6,001,130, both to Bryan, disclose polymer composite structures for cushioning intervertebral loads. The United States Patent Nos. 5,258,031 to Salib, et al. and 5,314,477 to Marnay disclose ball and socket type implants addressing the issue of intervertebral mobility. These patents are exemplary of a first approach using an elastomer as a motion and dampening structure and a second approach utilizing a ball and socket joint to create a moving pivot joint. There are many variations on these concepts, which include mechanical springs and more complex structural mechanisms. A significant portion of the prior art addresses the issues of intervertebral motion but do not address anatomical loading considerations.

The current state of prior art artificial intervertebral discs are associated with various problems. For example, a number of implants constructed from polymers are of insufficient strength to work effectively in the higher loading areas, such as the lumbar spine. Such polymers often take compressive sets so that the original height of the implant decreases over time. A surgeon must either compensate for the compression by initially using a larger polymer prosthesis and estimate compression or use the appropriately sized polymer prosthesis and later surgically replace the same once the irreversible compression of the prosthesis is unacceptable.

Implants constructed with ball and socket joints severely restrict or eliminate shock cushioning effect of a normal disc. This implant can provide motion, but biomechanically, the ball and socket joint negatively affects other healthy discs of the spine. The result can be long-term problems at other levels of the spine, as seen with the current treatment of fusion.

Other implants, not discussed above, utilize bearing surfaces usually having polyethylene bearing against metal interfaces. Polyethylene as a bearing surface is problematic in large joint replacement due to the wear properties of the material. Since artificial discs are intended to be implanted over long periods of time, such wear can be highly damaging to surrounding tissue and bone.

In view of the above, it is desirable to provide a solution to intervertebral disc replacement that restores motion to the damaged natural disc area while allowing for motion as well as cushioning and dampening, similar to the naturally occurring disc. In addition, it is preferable to allow such motion, cushioning, and dampening while preventing a polymer or elastomeric material from experiencing the relatively high compressive loads seen in the spine. It is also preferable to allow a bearing surface to share the spinal loads with the polymer and elastomeric material. Finally, it is preferable to control changes to the artificial motion intraoperatively to adjust for anatomical conditions.

According to the present invention, there is provided an artificial intervertebral disc including a housing having spaced inner surfaces facing each other and oppositely facing outer surfaces for engaging spaced apart intervertebral surfaces and a self-adjusting bearing operatively disposed between the inner surfaces of the housing for moving relative to the housing members to adjust and compensate for vertebral disc motion. Also provided is a mobile bearing including a self-adjusting bearing operatively disposed between the inner surfaces of the housing for moving relative to the housing members to adjust and compensate for movement of the housing. A method for posteriorly inserting an artificial disc assembly by inserting at least two artificial disc assemblies around a spine and into an intervertebral space.

Other advantages of the present invention can be readily appreciated as the same becomes better understood by reference to the following detailed description when considered in connection with the accompanying drawings wherein:
Figure 1 is a side perspective view of a preferred embodiment of the present invention;
Figure 2 is a side exploded view of the embodiment shown in Figure 1;
Figure 3 is a side perspective view of a second embodiment of the present invention;
Figure 4 is a perspective view of a lower disc constructed in accordance with the present invention;
Figure 5 is a side view of an upper disc constructed in accordance with the present invention;
Figure 6 is a top perspective view of an upper housing member made in accordance with the present invention;
Figure 7 is a top plan view of a lower housing member made in accordance with the present invention;
Figure 8 is a side perspective view of a third embodiment of the present invention;
Figure 9 is a perspective view of the present invention with the top housing member removed;
Figure 10 is a perspective view of an alternative pad configuration of the present invention;
Figure 11 is a perspective view of a further alternative embodiment of the pad member;
Figure 12 is a further alternative embodiment of the present invention;
Figure 13 is an exploded side perspective view of the embodiment shown in Figure 12;
Figure 14 shows an alternative embodiment of the housing members of the present invention;
Figure 15 shows a further alternative embodiment of the housing members of the present invention;
Figure 16 is an exploded view of a further embodiment of the present invention demonstrating a bayonet type locking of a disc member to a housing member;
Figure 17 is a perspective view of the disc member utilizing the bayonet locking mechanism to lock the disc member within a housing member;
Figure 18 is an exploded view of a disc member and housing member showing a further embodiment of a locking mechanism for locking the disc member within the housing member;
Figure 19 is a perspective view showing the disc member locked within the housing member;
Figure 20 is a perspective view of the a further embodiment of the housing member;
Figure 21 is a cross sectional view taken along line 21-21 in Figure 20;
Figure 22 is a perspective view of a load sharing pad member including flanges for locking engagement in the recesses of the housing member shown in Figures 20 and 21;
Figure 23 shows a further embodiment of a locking mechanism made in accordance with the present invention;
Figure 24 is a top view of the mobile bearing of the present invention;
Figure 25 is a top view of the artificial disc including a mobile bearing with no load sharing pads;
Figure 26 is a top view of the multidirectional mobile bearing of the present invention;
Figures 27A and B are side views of the mobile bearing of the present invention;
Figure 28 is a side perspective view of the mobile bearing of the present invention resting in a seat;
Figure 29 is a top perspective view of the seat and bearing combination in a housing having recesses for load sharing pads;
Figure 30 is a side perspective view of a third embodiment of the present invention;
Figure 31 is a perspective view of the base plate of a third embodiment of the present invention;
Figure 32 is a side view of a third embodiment of the lower housing of the present invention;
Figure 33 is a perspective view of the third embodiment of the present invention wherein a spherical surface is incorporated on the bearing;
Figure 34 is a perspective view of the third embodiment of the present invention wherein a spherical surface is incorporated on the bearing;
Figure 35 is a side view of the third embodiment of the present invention;
Figure 36 is a side view of the third embodiment of the present invention;
Figure 37 is a side perspective view of an alternative embodiment of the present invention;
Figure 38 is a perspective view of the base plate of the third embodiment of the present invention wherein the bearing is either convex or concave;
Figure 39 is a perspective view of the base plate of the third embodiment of the present invention wherein the bearing is either convex or concave; and
Figure 40 is a top perspective view of the bumpers of the present invention.

An artificial intervertebral disc constructed in accordance with the present invention is generally shown at 10 in the Figures. Like structures of various embodiments are indicated by primed numerals in the Figures. The invention is an artificial intervertebral disc, sometimes referred to by other terminology in the prior art such as intervertebral spacer device, or spinal disc for replacement of a damaged disc in the spine. The invention restores motion to the damaged natural disc that allows for motion as well as cushioning and dampening. As described below in more detail, the present invention also allows changes to the artificial disc motion intraoperatively to adjust for specific anatomical conditions.

Referring to the Figures, the disc 10 includes an upper housing member generally shown at 12 and a lower housing member generally shown at 14. The housing members 12, 14 include spaced inner surfaces 16 and 18 facing each other and oppositely facing outer surfaces 20, 22 for engaging spaced apart vertebral surfaces. A pair of bearing surfaces 24, 26 extend from each of the inner surfaces 16, 18 for engaging each other while allowing for low friction and compression resistant movement of the housing members 12, 14 relative to each other while under compression. As shown in the various Figures, the bearing surfaces are integral with disc members 28, 30. The housing members 12, 14 can be made from various materials including metals, such as titanium, as well as ceramics, and plastics. If integral with the bearing surfaces 24, 26, the housing members 12, 14 can be made from the preferred material for the bearing discs 28, 30 as discussed above. Based on this teaching, various other configurations can be made by those skilled in the art incorporating the present invention.

The upper and lower bearing surfaces 24, 26 engage each other when disposed correctly opposite each other. The configuration creates a three-dimensional bearing surface. As discussed below, the bearing surfaces 24, 26 are disposed on non-compressible discs or the like, thereby providing structure for absorbing compressive loads placed on the outer surfaces 20, 22 of the housing members 12, 14.

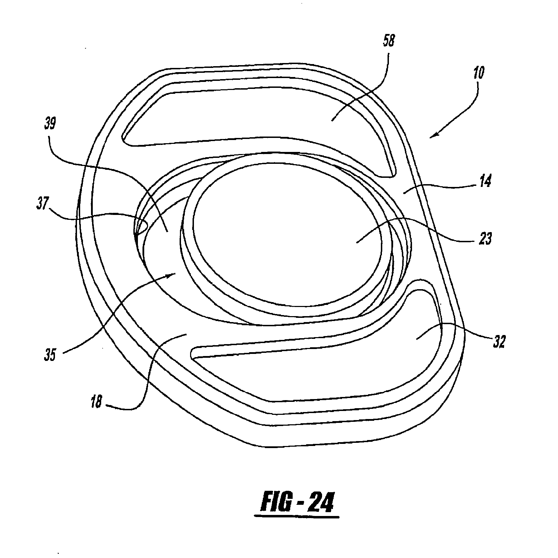

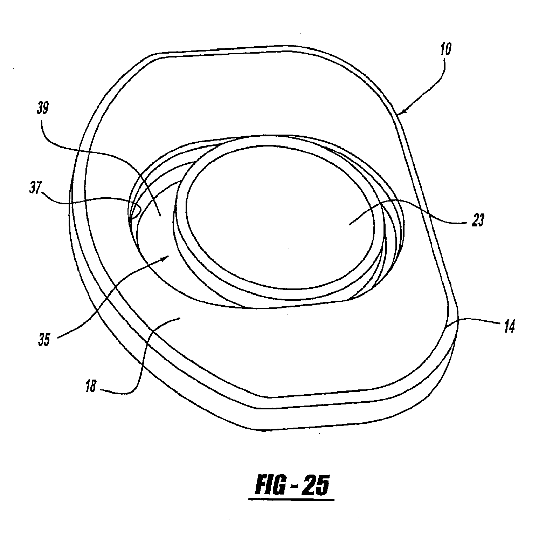

The bearing surfaces 24, 26 preferably form a mobile bearing 23 that is capable of automatically adjusting the position of the bearing 23 within a housing 14 as needed. The mobile bearing 23 is shown in Figures 24 through 29. The bearing 23 is preferably made of any material that slides along the surface of the housing 14 in which it is placed, with minimal to no wear, on either the bearing 23 or the housing 14. Examples of such materials include ceramic, metal, or other suitable materials that do not negatively react with the housing 14.

The bearing 23 of the present invention is disposed within a slot 35 of a housing 14. The bearing 23 is able to freely move or float within the slot 35 in response to movement of the housing 14. The bearing 23 is designed to provide proper cushioning and support of the housing 14 as is required by the specific system in which the bearing 23 is placed. The bearing can be used in any joint for providing proper support of the joint. For example, if the bearing 23 is used in an artificial intervertebral disc assembly, the bearing 23 provides cushioning so as to prevent the plates that are housing the disc from touching and wearing on one another. When the bearing 23 is utilized within the knee, the bearing also provides cushioning for the housing 14 during movement of the housing 14.

The bearing 23 disclosed herein can move freely under load conditions while maximizing the contact area of the upper and lower bearing surfaces 20, 24. In other words, within the slot 35 that the bearing 23 is disposed, the bearing 23 can move in any direction necessary to provide the proper support for the housing 14. The bearing 23 is able to move in this manner because the bearing 23 is a floating bearing, thus it is not attached or affixed to the housing 14 in which it is placed. Instead the bearing 23 "floats" within the housing 14, thus enabling the bearing 23 to be mobile and free to move in any direction necessary to provide proper support.

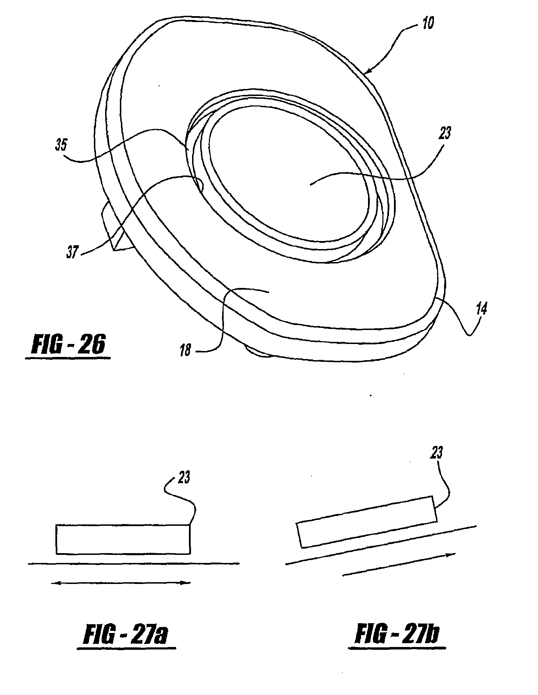

The housing 14 limits the "floating" motion of the bearing 23. In other words the movement of the bearing 23 can be limited based upon the size of the housing 14 and more specifically the slot 35 in which the bearing 23 is disposed. The slot 35 in which the bearing 23 is disposed dictates the range of movement of the bearing 23, i.e. movement can be constrained such that the bearing 23 can only move from an anterior to a posterior position. More specifically, the slot includes side walls 37, which define the size and shape of the slot 35, and a seat 39 on which the bearing is disposed. The movement of the bearing 23 is restricted based upon the shape of the walls 35 of the slot 35 in which the bearing 23 sits. For example, the slot 35 can be in the shape of a circle, an oval, or any other round-sided shape. The slot 35 must be shaped to have rounded sides so as to prevent the bearing 23 from lodging in a corner of the slot 35. The slot 35 can be formed such that the seat 39 does not have a uniform depth, such that there are peaks or angles within the slot 35, as shown in Figure 27. The lack of uniformity restricts movement of the bearing 23 within the slot 35 because the bearing 23 would require additional force in order to slide in the direction of the peak or angle.

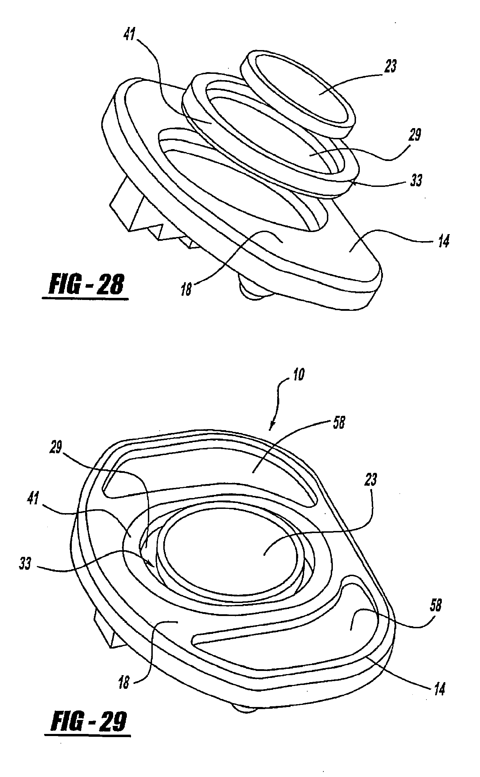

A removable insert 33, as shown in Figures 28 and 29, can also be disposed within the housing 14 for holding the bearing 23 in place. The insert 33 includes an upper surface 29 for engaging the bearing surfaces 24, 26. The insert 33, can be made of any material that enables the bearing 23 to functionally "float" across the insert 33 without excessive friction. The benefit of including the insert 33 in a housing 14 is that the insert 33 can be made of a different material than that of the housing 14. Accordingly, the housing 14 can be made from a first composition that is advantageous for the functionality of the housing and provides other strength characteristics while the insert 33 can be made from a more lubricious material to allow for more efficient friction-free movement of the bearing 23 thereon.

The movement of the bearing 23 is restricted based upon the shape of the insert 33 into which the bearing 23 is placed. The insert 33 includes side walls 41, which define the size and shape of the insert 33, and an insert seat 29 on which the bearing is disposed. The movement of the bearing 23 is restricted based upon the shape of the walls 41 of the insert 33 in which the bearing 23 sits. For example, the insert 33 can be in the shape of a circle, an oval, or any other round-sided shape. The insert 33 must be shaped to have rounded sides so as to prevent the bearing 23 from lodging in a corner of the insert 33. The insert 33 can be formed such that the insert seat 29 does not have a uniform depth, such that there are peaks or angles within the insert 33, as shown in Figure 27. The lack of uniformity restricts movement of the bearing 23 within the insert 33 because the bearing 23 would require additional force in order to slide in the direction of the peak or angle.

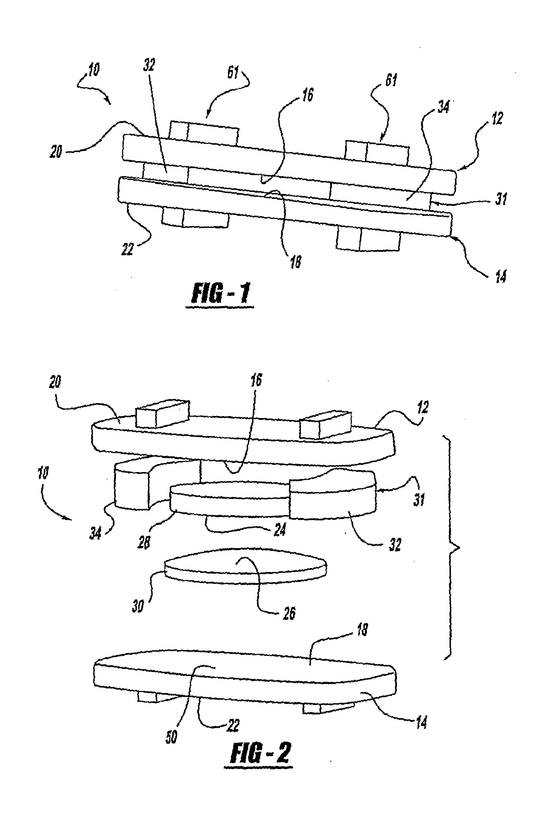

The housing 14 can also include load distributing dampening and cushioning pad recesses 32, 58. Load sharing pads 32, 34 generally shown at 31 and specifically indicated as pads 32 and 34 in Figures 1 and 2 are disposed between the inner surfaces 16, 18 and about at least a portion of the bearing surfaces 24, 26 for sharing absorption of compressive loads with the bearing surfaces 24, 26 while limiting relative movement of the housing members 12, 14. More specifically, under in vivo loading conditions, the centralized bearing surfaces 24, 26 and the floating bearing surfaces not only provide for three-dimensional movement relatively between the housing members 12, 14, but also share with the load sharing pads 32, 34 the function of distributing compressive loads on the device 10 to provide a system for motion and effective load distribution. The centralized low friction and compression resistant bearing surfaces 24, 26 allow full motion in multiple planes of the spine while the load distributing damper and cushioning pads 32, 34 simultaneously share the load. Critical is the function of the pads 32, 34 sharing the load with the bearing surfaces 24, 26. Although the pads 32, 34 can be compressible, the compression is limited by the noncompressibility of the bearing surfaces 24, 26. Likewise, although the bearing surfaces allow for motion in multiple planes, the pads 32, 34 are fixedly secured to the housing members 12, 14, thereby allowing for a degree of flexibility and therefore movement of the housing members 12, 14 relative to each other, yet limiting such movement. In total, each element, the bearing surfaces 24, 26, and pads 32, 34, allow for movement, yet limit such movement, whether it is the sliding movement of the bearing surfaces 24, 26 or the cushioning movement allowed by the pads 32, 34. Each element allows for relative movement, yet each element limits the movement of the other element of the system.

In view of the above, the system allows restoration of normal motion while maintaining load cushioning capabilities of a healthy disc. This is particularly apparent with motion of the spine. Any rotation of the upper and lower housing members 12, 14 causes the load distributing dampening and cushioning pads 32, 34 to absorb some of the load.

As shown in the various Figures, the bearing surfaces 24, 26 can include a concave surface portion on one of the upper or lower disc members 28, 30, and a convex surface portion on the other. The concave surface is seated within the convex surface for sliding movement relative thereto effectively resulting in relative pivoting motion of the housing members 12, 14, which compresses at least a portion of the load sharing pads 32, 34 while extending at least a portion of the oppositely disposed load bearing pad 32, 34. Alternatively, either one of the top and bottom disc members 28, 30 can have either of the convex or concave surfaces.

The disc members 28, 30 can be made from a composition that is noncompressible. Such compositions can be selected from the group including ceramics, plastics, and metal bearing materials, such as cobalt and chrome. Alternatively, the housing members 12, 14 can include projections wherein the disc members 28, 30 are effectively integral with the housing members 12, 14. In this situation, the entire housing, including the projections having the bearing surfaces 24, 26 thereon, can be made from the noncompressible material, preferably a ceramic. As stated above, alternative configurations can be made by those skilled in the art once understanding the present invention.

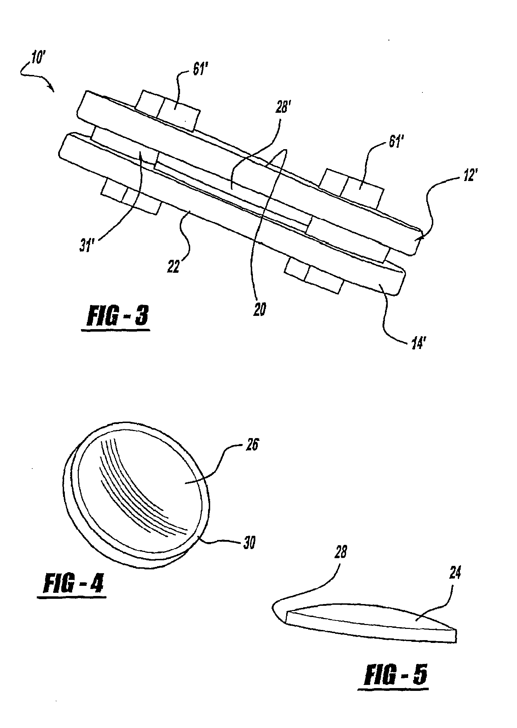

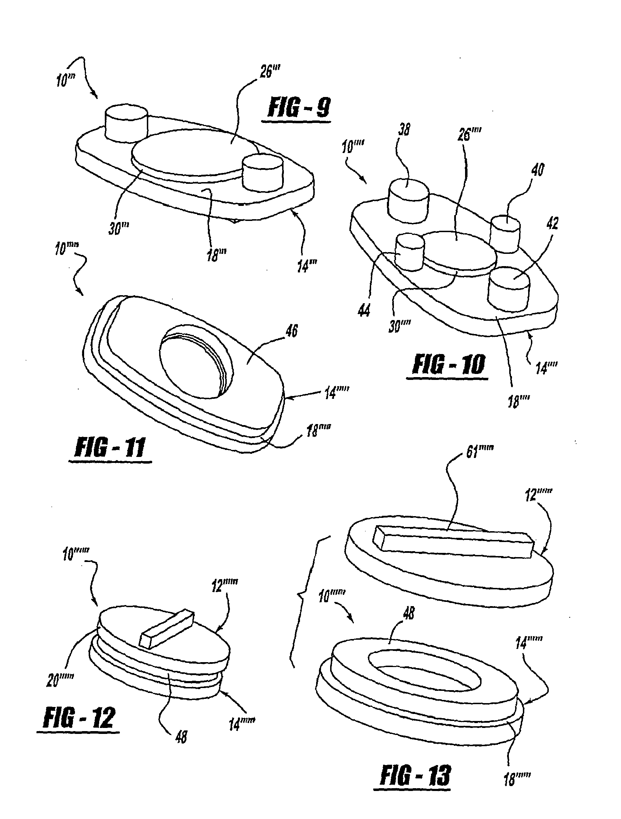

The load sharing pads 32, 34 can be in various configurations shown in the Figures, such as paired pads 32, 34 shown in Figures 1-3. Alternatively, the device 10 can include four oppositely disposed pads 38, 40, 42, 44 as shown in Figure 10. A further embodiment of the invention is shown in Figure 11, wherein a single pad 46 substantially covers the surface 18""' of the housing member 14""'. The pads can contour to the shape of the housing members such as shown in Figures 12, 13, wherein the pad member 48 is an annular pad member disposed with a annular housing 12"""; 14""". The selection of such housing members 12, 14 and pad members 31 can be determined based on the location of the placement of the device 10 as well as the spacing conditions between the vertebrae and load bearing necessities depending on the level of the spine being addressed. In other words, different shaped devices, such as the round shaped housing members shown in Figure 12 can be used for placement between smaller discs, such as cervical spines whereas more rectangular shapes, such as the housing members shown in Figures 1-11 can be used in between lumbar vertebrae.

The load sharing pads 31, in which ever shape they are configured, are elastic for allowing relative twisting movement between the housing members 12, 14 effecting relative three-dimensional movement between the housing members 12, 14, while limiting the movement and preventing contact between the housing members 12, 14 except for the contact between the bearing surfaces 24, 26. By elastic, it is meant that the pad members 31 are compressible and stretchable, yet provide a self-centering effect on the assembly with specific regard to the housing members 12, 14, as well as the bearing surfaces 24, 26. Deflection or rotation of the forces created due to relative movement of the bearing surfaces 24, 26, and likewise the housing members 12, 14, forces the pads 31 to act in such a way to counter the force, thus allowing a unique self-centering capability to the assembly 10. While in an ideal situation, wherein the patient's facets are uncompromised and ligamental balances are intact, this self-centering aspect may not be completely necessary. In other words, the patient's anatomy may still provide stabilization and specifically, ligaments may provide self-centering. However, ligamental imbalance, and damaged facets would normally make an artificial disc questionable, at best, with use of the current technology that is available. In such cases, having the ability to self-center and restrict motion (the pads 31 of the present invention are elastic and thus restrict motion by stretching and returning to rest), the possibility of extending indications to patients currently considered outside of the scope of artificial disc technology will be highly advantageous.

The pads 31 of the present invention provide further advantages to the invention. A key advantage is the ability to adjust the pads 31 to patient and surgeon requirements. In such cases wherein range of motion needs to be restricted due to compromised facets, a harder, less elastic pad can be inserted between the housing members 12, 14. Since this less elastic pad would move and stretch less, the disc would be automatically restricted in motion. This method of adjusting pads can be done intraoperatively to compensate for surgical and patient conditions. To one skilled in the art, one can fine-tune the assembly 10 to a patient and surgeon's needs with multiple pads of different properties or materials.

The pads 31 are made from a polymer or elastomer that allows deflection under load. Examples of such polymers and elastomers are silicone, polyurethane, and urethane composites. As discussed above with regard to flexibility or elasticity, the content and composition of the pads 31 are adjustable. A highly dense material creates a very rigid disc, while a very soft material creates a very free moving disc. The motion would be restricted in all planes of the pad depending upon these factors. Rotation is also restricted, as well as flexion or movement of the disc. The amount of compression possible is restricted or allowed according to the pads material properties. This is true of motion towards the back or side-to-side motion. Thus, the pads 31 are always in contact and always share the load, under any adjustment of relative positioning of the housing members 12, 14. Since motion forces the pads to be in contact, the pads 31 automatically damper loads imposed by the artificial disc construct 10.

With specific regard to the flexibility or elasticity of the polymer or elastomer composition of the pads 31, the pads can be selected from a composition having a durometer from 20 to 98 on the Shore OO Scale. Alternatively, the pads 31 can be selected from a composition having a durometer from 10 to 100 on the Shore A Scale. A further alternative is for the pads 31 to be selected from a composition having a durometer from 22 to 75 on the Shore D Scale. In any event, the pad members 31 can be selected during the operation and procedure by the clinician to suit a specific situation. Although the pad members 31 can be pre-inserted between the housing members 12, 14 prior to insertion of the device 10 in situ, the various configurations of the present invention can allow for in situ replacement of the pad members 31 so as to custom select the flexibility or elasticity of the members. In this manner, the pad members 31 are custom designed for the individual environment of the intervertebral space into which the device is being disposed.

The disc members 28 and 30, and pads 31 can be contained or locked in position in between the housing members 12, 14 by various means. The disc 28, 30 can be locked to the housing members 12, 14 by a press fit taper, retaining ring, or other means. The key aspect of such locking mechanisms is to prevent the disc members 28, 30 from moving against the upper or lower housing members 12, 14 once installed in order to prevent additional wear.

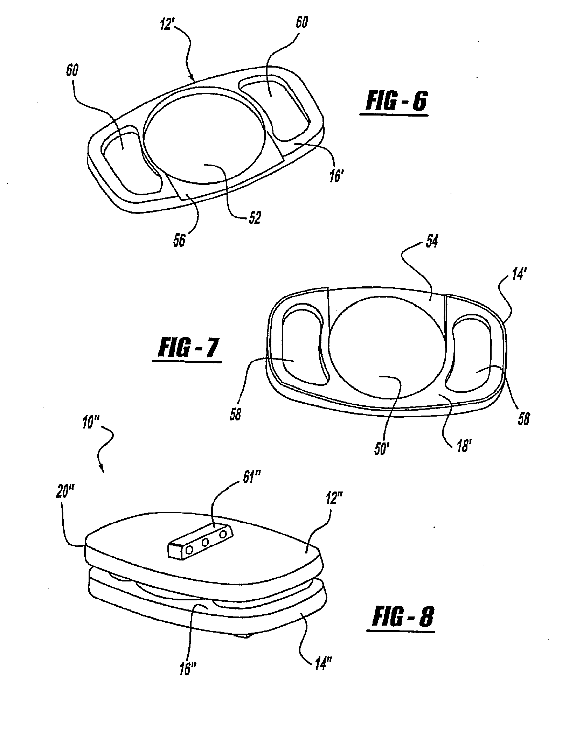

Figures 1 and 2 show disc members 28, 30 disposed in recesses (only the lower recess 50 is shown in Figure 2 in an exploded view) in each of the inner surfaces 16, 18 of the housing members 12, 14. Figures 6 and 7 show plan views of a second embodiment of the housing member 12', 14', wherein each recess 50', 52 includes a ramped surface 54, 56 leading from an outer edge to the inwardly tapered recess portion 50', 52. The ramping 54, 56 allows access of the disc members 28,30 in between the housing members 12', 14' after placement of the housing members 12', 14' in the intervertebral space. This intraoperative access of the disc members 28, 30 allows the surgeon to test different size disc members under load conditions to perfectly fit the disc members in place. Such an advantage is not obtainable with any prior art device.

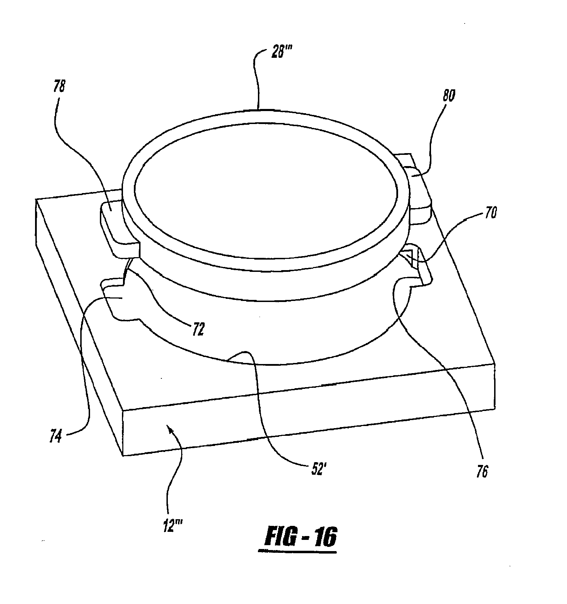

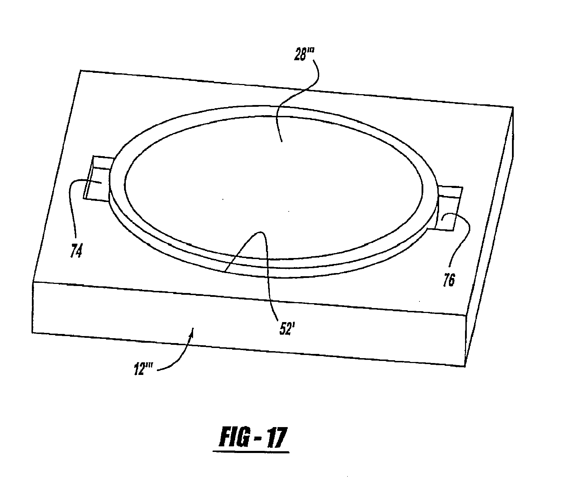

An alternative mechanical mechanism for locking the disc members within the housing members are shown in Figure 16. The representative housing member 12"' includes recess 52'. The recess 52' includes a substantially arcuate peripheral undergroove 70. The groove is defined by a lip portion 72 including at least one and preferably at least two openings 74, 76. The disc member 28"' includes bayonet style flanges 78, 80 extended radially outwardly therefrom, the flanges 78, 80 being shaped so as to be received through recess 74, 76. In operation the disc member 28"' can be disposed within the recess 52' such that the flanges 78, 80 align with recesses 74, 76. Once the disc member 28"' can be rotated thereby providing a bayonet style locking mechanism of the disc member 28"' within the housing 12"', as shown in Figure 17.

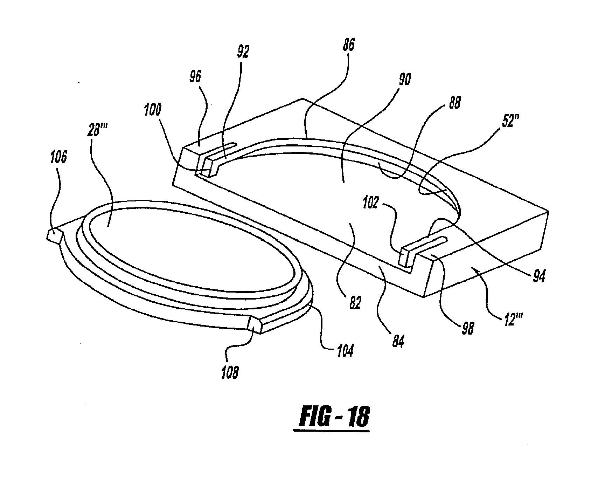

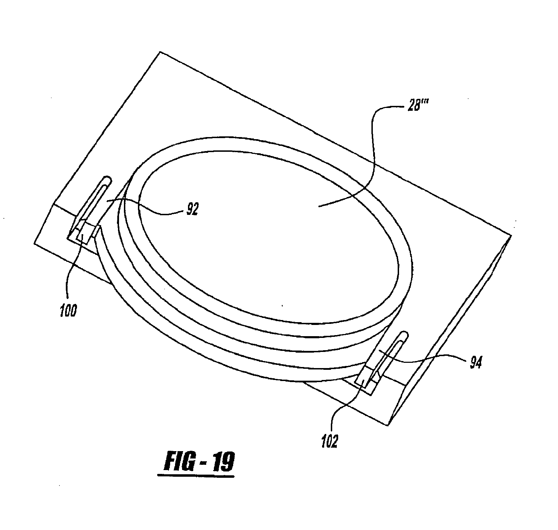

A further alternative embodiment of the locking mechanism is shown in Figures 18 and 19. The housing member 12"' includes a substantially arcuate recess 52" having an open end portion 82 extending to an edge 84 of the housing member 12"'. The recess 52" includes a lip portion 86 extending about a substantial portion thereof defining an inner groove 88 between the seating surface 90 of the recess 52" and the lip portion 86. Arm portions 92, 94 are extensions of the lip portion 86 but extend from and are separate from peripheral ends 96, 98 of the housing member 12"'. The arm portions 92, 94 have a spring-like quality such that they can be deflected outwardly from the arcuate circle defined by the recess 52". Each of the arms 92, 94 has an elbow portion 100, 102 extending from each arm portion 92, 94 towards the seating surface 90, respectively. The disc member 28"' includes a substantially arcuate peripheral, radially outwardly extending flange portion 104. The flange portion 104 includes two abutment edges 106, 108. In operation, the flange 104 and disc member 28"' are disposed within the annular recess or groove 88, deflecting outwardly the arms 92, 94. Once disposed in the recess 52", as shown in Figure 19, the elbows 100, 102 engage the abutment surfaces 106, 108 of the disc member 28"' thereby locking the disc member 28"' in place. Outward deflection of the arms 92, 94 can selectively release the disc member 28"' from locked engagement to provide for further adjustment of the selection of the disc member during an operation procedure.

Also, as best shown in Figures 6 and 7, the pads members 31 can be disposed in recesses 58, 60 in the lower and upper housing members 12', 14' respectively. It is preferable to permanently adhere the pad members 31 to the housing members 12', 14' by use of mechanical mechanisms and/or various adhesives, such as cyanoarylates, urethanes, and other medical grade adhesives. This list of adhesives, as with other listings of ingredients in the present application, is merely exemplary and not meant to be exhaustive.

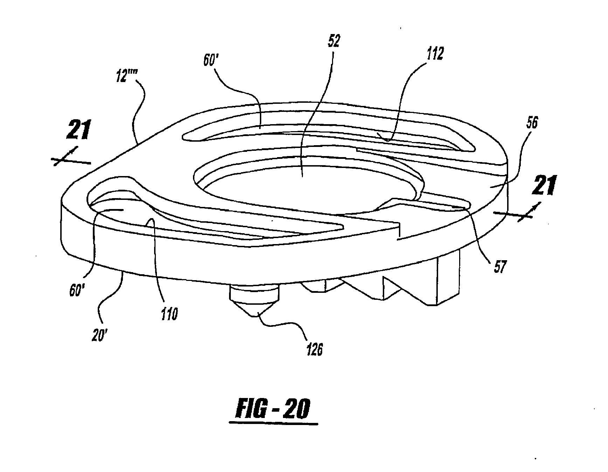

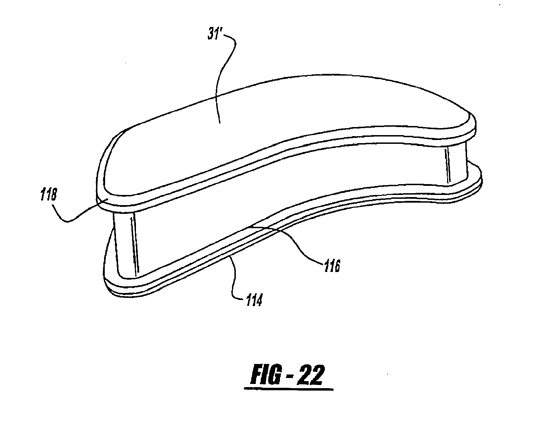

Examples of mechanical mechanisms for locking the pad members 31 into recesses in the housing members are shown in Figures 20-23. One such mechanism is an undercut locking mechanism shown in Figures 20 - 22. Housing member 12"" includes a central recess 52 such as shown in Figure 6 having a ramp portion 56. The ramp portion 56 includes a centrally located tongue groove 57 allowing for the insertion of a spatula type device under a disc member disposed within the recess 52 for releasing the disc member from the recess, similar to the use of a shoehorn type mechanism. Recesses 60' include undercut recesses 110, 112 for locking engagement with a peripheral flange portion 114 extending from an edge 116 of a pad member 31'. Since the pad member is made from a deflectable material, the flange portion 114 can be force-fit into and seated within the undercut 110, 112. The undercut locking mechanism effectively prevents the pad member 31' from disengagement with the housing member 12"" in situ. Of course, the upper flange 118 would be locked within a similar undercut locking detail of recesses within the opposing housing member (not shown).

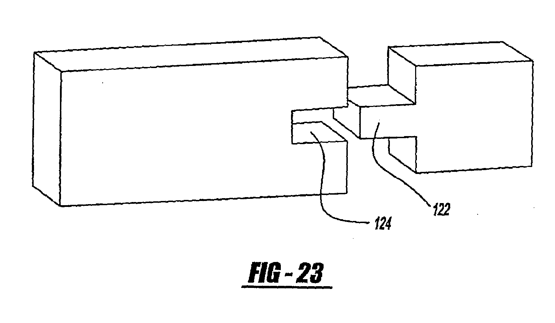

An alternative locking mechanism between the pad member and housing member can be a tongue-and-groove relationship as shown in Figure 23. Either the pad or the housing can include the tongue portion 122 and the other pad and housing members can include the groove 124. In other words, either of the locking members can include the tongue 122 and the other of the members being locked would include the groove 124. An alternative of this or the other locking mechanism shown is that the recess and/or pad can include multiple grooves or slots as well as multiple tongues.

The various recesses or pockets 50', 52, 58, 60 can be of different relative sizes and shapes. For example, the upper housing member 12' may have a larger recess or pocket for seating a relatively larger one of said discs 28 and the lower housing member 14' may be include a smaller (larger and smaller referring to diameter of the annular recess) of the recesses or pockets for seating a relatively smaller one of the lower disc 30, thereby providing for an increased range of motion at the bearing surface interface.

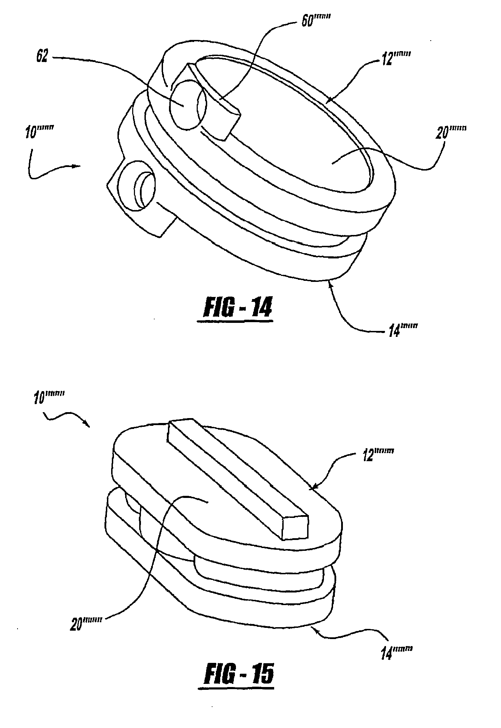

The various Figures show that the outer surfaces 20, 22 of the various embodiments of the housing members 12, 14 can include flanges generally indicated at 60. The flanges 60 or fins, as they are sometimes referred to in the art, provide a mechanism for fixation to the intervertebral surfaces. Various embodiments, such as those shown in Figures 1 and 2 are dual fin constructs. Other embodiments, such as those shown in Figures 8, 12, and 13 are single fin or single flange constructs. Depending upon the nature of the surfaces to which the outer surfaces 20, 22 are to abut, the surgeon can select various flange or fin configurations. Additionally, the fins 60 can be located in alternative positions, either centrally as shown in many of the Figures, or peripherally, as shown in Figure 14, for a specific use with anterior extension plates, as with screw fixations. The flanges, such as flange 60"""' can include a bore 62 therethrough, which can be either a smooth surface or threaded depending on its intended use.

The outer surfaces 20, 22 can be smooth, which allows for easier revision as it allows for minimal to no ingrowth or they can be textured. Texturing of the outer surfaces 20, 22 allows ingrowth for long-term fixation of the assembly 10. Porous coatings, plasma spray, grit blasting, machining, chemical etching, or milling are examples of techniques for creating ingrowth capable surfaces. Coatings that enhance bone growth can also be applied. Examples of such coatings are hyroxyapatite and bone morphogenic proteins.

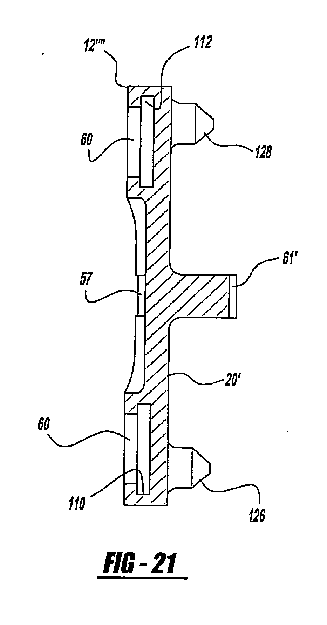

Figures 20 and 21 provide structure for further rotational stability of the device in situ. The housing member 12"" includes pointed portions 126, 128 extending from the outer surface 20' thereof. The point members 126, 128 function in conjunction with the flange portion 61' to engage an opposing vertebral surface. The point portions 126, 128 being disposed radially peripherally from the centrally disposed flange 61' provide at least a three-point engagement of the vertebral surface thereby preventing rotation of the housing member 12"" relative thereto. Of course, the point portions 126, 128 can be in made in various configurations and extend various amounts from the outer surface 20' to' be custom suited to a specific vertebrae surface shape.

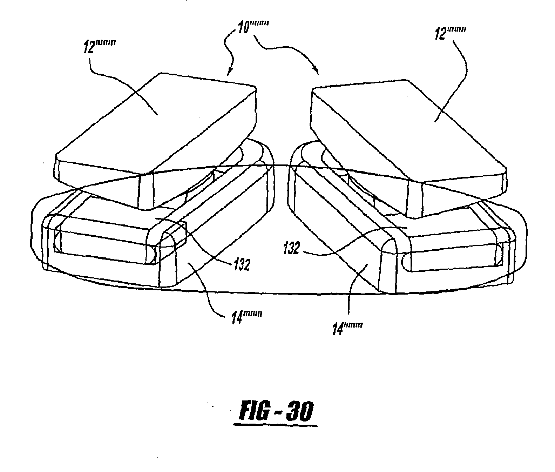

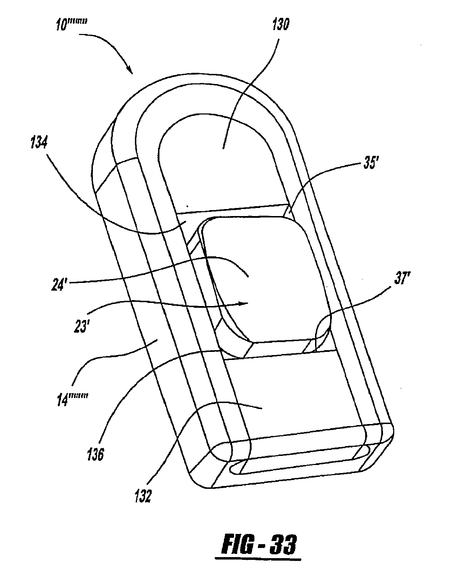

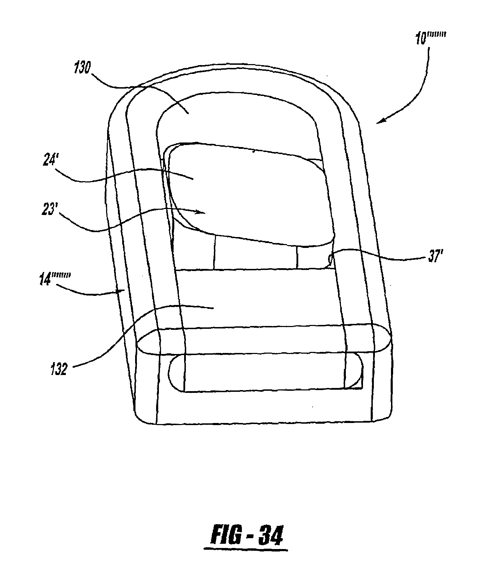

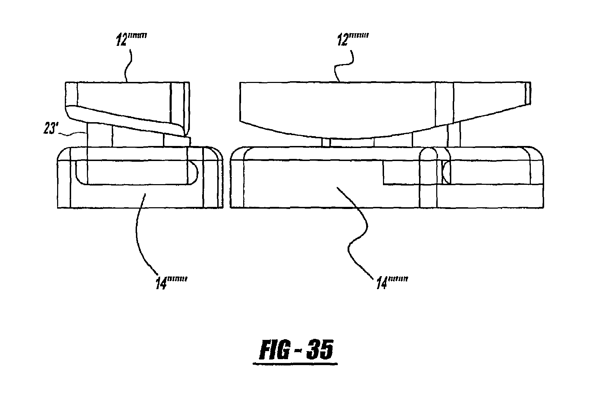

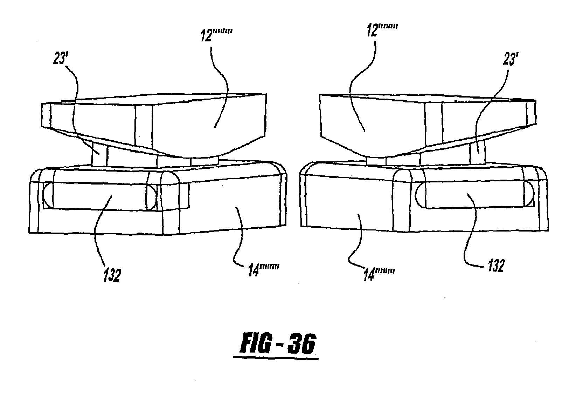

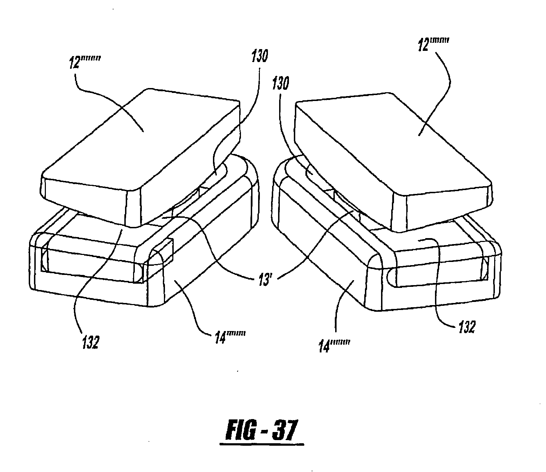

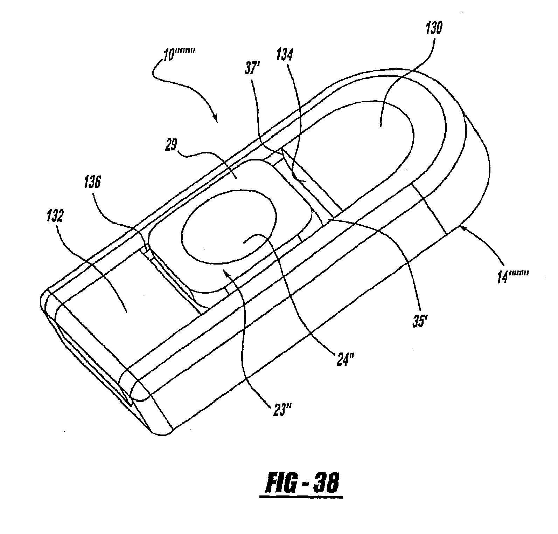

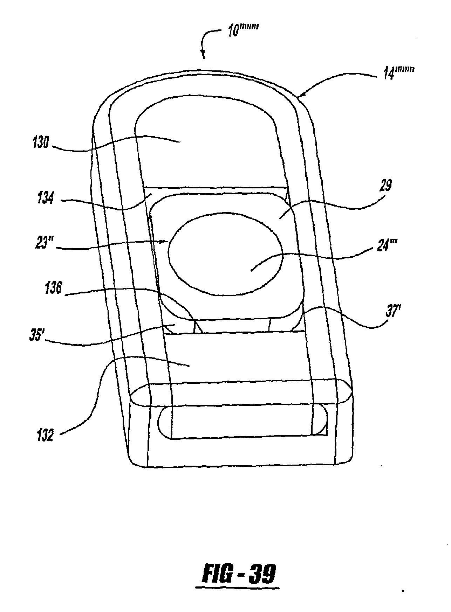

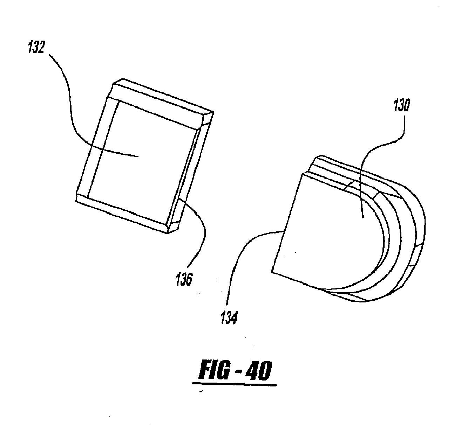

Alternatively, as shown in Figures 30-40, the disc 10"""" can be formed as two separate pieces that are inserted into an intervertebral space, generally shown as 146 in Figure 30. The benefit of this formation of the disc 10"""" is that the discs 10"""" can be inserted during a posterior insertion. The two discs 10"""" function so that the units work in tandem and effectively become one artificial disc assembly. The arrangement of the two discs 10"""" enables each disc 10"""" to be inserted on either side of the spinal column into the intervertebral space 146 and work in conjunction as a single artificial disc assembly 10"""". The two discs 10""""are angled toward the mid-line of the vertebral body 146. While two disc assemblies 10"""" are described herein, more than two discs 10"""" can also be utilized without departing from the spirit of the present invention.

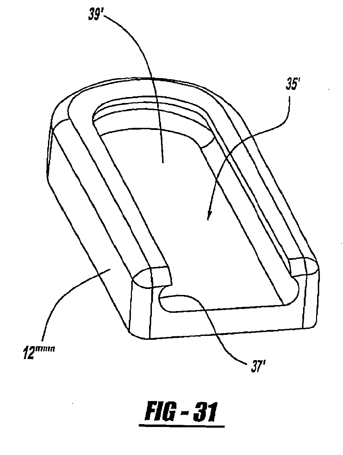

Each of the discs 10"""" include an upper housing member 12"""" and a lower housing member 14"""". The housing members 12"""", 14"""" each include a slot 35' within the housing member 12"""", 14"""". The slot 35' enables the bearing 23 to move freely or "float" within the slot 35' in response to movement of the housing 14. As shown in Figures 31, 33-34, and 38-39, the slot 35' can be formed in any shape that enables proper movement of the bearing 23, however, preferably the slot 35' is an open-ended u-shaped slot with a seat 39' and side walls 37'. The side walls 37' maintain the bearing 23 in proper alignment within the housing 12"""", 14"""". As disclosed above, the bearing 23 is capable of floating within the slot 35', thus enabling the bearing 23 to be mobile and free to move in any direction necessary to provide proper support for the housing 12"""", 14"""". The housing 12"""", 14"""" limits the motion of the bearing 23. The size of the housing 12"""", 14"""" and, more specifically, the slot 35' in which the bearing 23 is disposed limits the motion of the bearing 23. Further, bumpers 130, 132 can also be included in the slot 35' to further limit the motion of the bearing 23, provide dampening of the motion of the bearing 23 and prevent the bearing from being displaced from the housing 12"""", 14"""". The bumpers 130, 132 can be of any size sufficient to provide the necessary limitations on the bearing 23. For example, a single bumper can be used for both housings 12"""", 14"""". Alternatively, each housing 12"""", 14"""" can incorporate separate bumpers 130,132. The bumpers 130,132 are also useful for load sharing and thereby preventing the housing members 12"""", 14"""" from contacting one another. The bumpers of the present invention 130, 132 are shaped to conform to the shape of the slot 35'. In other words, the bumpers 130, 132 are shaped to precisely fit the slot 35' in which the bumpers 103, 132 are displaced. Preferably, the bumpers 130, 132 do not extend beyond the length of the housing 12"""", 14"""". The bumpers 130, 132 have walls 134, 136 respectively that engage the wall 37' of the slot 35'. This enables the bumpers 130, 132 to be maintained in alignment and prevents the bumpers 130, 132 from moving.

The upper housing 12"""" can either Include a slot 35' identical to that of the lower housing 14"""" or can include a single piece having a matching bearing that complements that of the bearing 23. In other words, the upper housing 12"""" can either have a slot 35' that is identical to the shape of the slot 35' of the lower housing 14"""", such that the bearing 23 moves both in both housings 12"""", 14"""" equally or the upper housing 12"""" can be formed such that only a single piece is utilized and there is no movement within the top plate of the bearing 23.

The bearing 23' includes side arms 138, 140 that slidably engaged the wall 37' of the slot 35'. The bearing 23' is therefore held in position within the slot 35' via the side arms 138, 140 and the bumpers 130, 132.

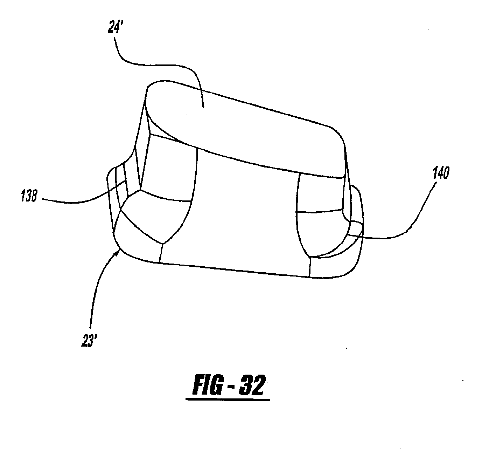

The bearing 23 of the present invention can also have incorporated on the bearing surface 24 various shapes as shown in the figures. Specifically, Figure 32 shows the bearing surface 24', wherein the surface 24' is a spherical surface. The spherical surface 24' enables the center of rotation of the bearing 23' to exist at the center of the sphere. Therefore, the pair of discs 10"""" functions as a single artificial disc with one center of rotation. Alternatively, the bearing 23 can have a surface that is either convex 24" or concave 24"'. This embodiment is specifically shown in Figures 9 and 10 wherein the center portion of the bearing 23' is either convex or concave and there is a flat portion 29 of the bearing 23'. When a convex or concave surface 24", 24"' respectively, is utilized, the rotation center is not in the center for side-to-side rotation. Thus, the assembly is somewhat resistant to side-to-side bending but is more easily aligned.

The housings 12"""", 14"""" can be inserted simultaneously without incorporating the floating bearing 23 initially. This enables the disc 10"""" to be inserted into the intervertebral space and once the disc 10"""" has been inserted, the bumpers 130, 132 and the bearing 23 can be slid into place within the slot 35'.

Various methods can be utilized for insertion of the present invention in situ. For example, an assembled device 10 as shown in Figure 1, can be disposed between the intervertebral spaces during surgery, after calculation of space, depth, and height. Alternatively, opposing housing members 12, 14 can be disposed between the intervertebral spaces and pads 31 and disc members 24, 26 can be tested in situ prior to fixation thereof to allow for custom sizing. Accordingly, the present invention broadly provides a method of assembling an artificial intervertebral disc 10 in vivo by inserting upper and lower housing members 12, 14 into an intervertebral space and disposing cushioning pads 31 between the inner surfaces 16, 18 of the housing members 12, 14, thereby placing the pads in compression. The pair of disc members 28, 30 are inserted between the inner surfaces of the plates 16, 18. The disc members 28, 30 have abutting low friction surfaces 24, 26 therebetween. The disc members 28, 30 are surrounded by the pads 31, whereby the disc members 28, 30 and pads 31 are under compressive forces and share such compressive forces. This step of the bearing surfaces 24, 26 and shock absorbing pads 31 sharing absorption of the compressive forces and limiting the relative movement of the housing members 12, 14 is an advantage not found in the prior art.

One use of the bearing of the present invention is in an artificial intervertebral disc for replacement of a damaged disc in the spine. The artificial disc 10 of the present application includes a mobile bearing 23 that allows for the bearing 23 to move to adjust and compensate for vertebral disc motion. By permitting the bearing to self-adjust, the bearing 23 can more freely move under translation loading conditions while maximizing the contact area of the upper and lower bearing surfaces 20, 24.

In applications such as the lumbar spine, the disc upper member and lower member are angled relative to each other to maintain spinal curvature. The load distributing damper and cushioning pads are always under some load when the spine is moving, although they can be adjusted for a neutral no load situation when the spine is not moving.

The load distributing damper and cushioning pads also create an elastic means of self-centering the disc construct. Deflection of rotation of the disc forces the pads to act in such a way as to counter the force, thus allowing a unique self-centering capability. In an ideal situation where the patient's facets are uncompromised and ligamental balance is intact, this is not necessary. However, ligamental balance and damaged facets would normally make an artificial disc questionable at best with the current art. In such cases, having the ability to self-center and restrict motion (the pads are elastic and thus restrict motion by stretching and returning to rest), the possibilities of extending indications to patients currently considered outside the scope of artificial disc technology is highly advantageous. In a floating bearing design, the ability to self-center mixed with the dampening abilities of the pads creates an ideal system for an artificial disc.

The pads can also be adjusted according to patient and surgeon requirements. In such cases where range of motion needs to be restricted due to compromised facets, a harder, less elastic pad can be inserted. Since a less elastic pad moves and stretches less, the disc is automatically restricted in motion. This method of adjusting pads can be done interoperatively to compensate for surgical and patient conditions.

Throughout this application, various publications, including United States patents, are referenced by author and year and patents by number. Full citations for the publications are listed below. The disclosures of these publications and patents in their entireties are hereby incorporated by reference into this application in order to more fully describe the state of the art to which this invention pertains.

The invention has been described in an illustrative manner, and it is to be understood that the terminology that has been used is intended to be in the nature of words of description rather than of limitation.

Obviously, many modifications and variations of the present invention are possible in light of the above teachings. It is, therefore, to be understood that within the scope of the appended claims, the invention can be practiced otherwise than as specifically described.

Further aspects of the invention can be found in the following paragraphs:
1. An artificial intervertebral disc comprising:
   housing members including spaced inner surfaces facing each other and oppositely facing outer surfaces for engaging spaced apart intervertebral surfaces; and
   self-adjusting bearing means operatively disposed between said inner surfaces for moving relative to said housing members to adjust and compensate for vertebral disc motion.
2. The disc as described in paragraph 1, wherein said inner surfaces include at least one slot within each of said inner surfaces for seating said self-adjusting bearing means therein and allowing movement of said self-adjusting bearing means.
3. The disc as described in paragraph 2, wherein said slot includes outer walls defining the size of said slot and receiving means inside said slot, said receiving means for receiving and containing said bearing means therein.
4. The disc as described in paragraph 3, wherein said receiving means includes a seat portion integral within said housing.
5. The disc as described in paragraph 2, wherein said slot is in a shape selected from the group consisting essentially of a circle, an oval, and other round-sided shapes.
6. The disc as described in paragraph 1, wherein said housing members are constructed from a composition selected from the group consisting essentially of metals, ceramics, and plastics.
7. The disc as described in paragraph 6, wherein said housing members include an outer surface having a surface texture for accepting bone growth therein.
8. The disc as described in paragraph 7, wherein said surface texture is selected from the group consisting essentially of physically roughened, porous coated, and plasma coated surfaces.
9. The disc as described in paragraph 3, wherein said receiving means is a removable insert.
10. The disc as described in paragraph 9, wherein said insert includes outer walls defining a size of said insert and a seat for receiving and containing said bearing means therein.
11. The disc as described in paragraph 10, wherein said insert is in a shape selected from the group consisting essentially of a circle, an oval, and other round-sided shapes.
12. The disc as described in paragraph 9, wherein said insert is constructed from a composition selected from the group consisting essentially of metals, ceramics, and plastics.
13. The disc as described in paragraph 1, wherein said bearing means is constructed from a composition selected from the group consisting essentially of metals, ceramics, and plastics.
14. A mobile bearing comprising self-adjusting bearing means operatively disposed between inner surfaces of a housing for moving relative to said housing to adjust and compensate for motion of the housing.
15. The mobile bearing as described in paragraph 14, wherein said inner surfaces include at least one slot within each of said inner surfaces for seating said self-adjusting bearing means therein and allowing movement of said self-adjusting bearing means.
16. The mobile bearing as described in paragraph 15, wherein said slot includes outer walls defining a size of said slot and receiving means inside said slot for receiving and containing said bearing means therein.
17. The mobile bearing as described in paragraph 16, wherein said receiving means includes a seat portion integral with said housing.
18. The mobile bearing as described in paragraph 15, wherein said slot is in a shape selected from the group consisting essentially of a circle, an oval, and other round-sided shapes.
19. The mobile bearing as described in paragraph 16, wherein said receiving means is a removable insert.
20. The mobile bearing as described in paragraph 19, wherein said insert includes outer walls defining a size of said insert and a seat portion integral with said insert for containing said bearing means therein.
21. The mobile bearing as described in paragraph 20, wherein said insert is in a shape selected from the group consisting essentially of a circle, an oval, and other round-sided shapes.
22. The mobile bearing as described in paragraph 19, wherein said insert is constructed from a composition selected from the group consisting essentially of metals, ceramics, and plastics.
23. The mobile bearing as described in paragraph 14, wherein said bearing means is constructed from a composition selected from the group consisting essentially of metals, ceramics, and plastics.
24. The mobile bearing as described in paragraph 14, further including load sharing means disposed between said inner surfaces and about at least a portion of said bearing means for sharing absorption of compressive loads with said bearing means while limiting the relative movement of said housing.
25. A method of automatically adjusting support of a housing by floating a mobile bearing within the housing for automatically adjusting for motion of the housing and providing support relative to the motion.
26. The method as described in paragraph 25, wherein said floating step includes moving the bearing into a slot within the housing.
27. The method as described in paragraph 26, further including limiting the motion of the bearing by altering the dimensions of the slot to conform to the desired range of motion.
28. An artificial intervertebral disc comprising:
   housing members including spaced inner surfaces facing each other and oppositely facing outer surfaces for engaging spaced apart intervertebral surfaces;
   self-adjusting bearing means operatively disposed between said inner surfaces for moving relative to said housing members to adjust and compensate for vertebral disc motion;
   self-centering means for automatically centering said self-adjusting bearing means within said housing members.
29. A method of adjusting support of a housing by centering a mobile bearing within the housing for automatically adjusting for motion of the housing and providing support relative to the motion.
30. An artificial joint comprising:
   housing members including spaced inner surfaces facing each other and oppositely facing outer surfaces for engaging spaced apart intervertebral surfaces;
   self-adjusting bearing means operatively disposed between said inner surfaces for moving relative to said housing members to adjust and compensate for vertebral disc motion; and
   self-centering means for automatically centering said self-adjusting bearing means within said housing members.
31. The joint as described in paragraph 30, wherein said self-centering means are load sharing means disposed between said inner surfaces and about at least a portion of said bearing means for sharing absorption of compressive loads with said bearing means while limiting the relative movement of said housing members and self-centering said bearing means.
32. An artificial intervertebral disc comprising:
   housing members including spaced inner surfaces facing each other and oppositely facing outer surfaces for engaging spaced apart intervertebral surfaces;
   self-adjusting bearing means operatively disposed between said inner surfaces for moving relative to said housing members to adjust and compensate for vertebral disc motion; and
   said inner surfaces include at least one slot within each of said inner surfaces for seating said self-adjusting bearing means therein and allowing movement of said self-adjusting bearing means.
33. The disc as described in paragraph 32, wherein said bearing includes a convex surface.
34. The disc as described in paragraph 32, wherein said bearing includes a concave surface.
35. The disc as described in paragraph 32, wherein said bearing includes a spherical surface.
36. The disc as described in paragraph 32, wherein said bearing means includes arm means for maintaining said bearing within said slot.
37. A method for posteriorly inserting an artificial disc assembly by inserting at least two artificial disc assemblies around a spine and into an intervertebral space.
38. The method as described in paragraph 37, wherein said inserting step includes inserting each of the assemblies about a side of the spine into opposite sides of the intervertebral space.

## Claims

1. An artificial intervertebral disc comprising:
housing members including spaced inner surfaces facing each other and oppositely facing outer surfaces for engaging spaced apart intervertebral surfaces; and
self adjusting bearing means operatively disposed between said inner surfaces for moving relative to said housing members to adjust and compensate for vertertebral disc motion, further including load sharing means disposed between said inner surfaces and about at least a portion of said bearing means for sharing absorption of compressive loads with said baring means while limiting the relative movement of said housing members.

2. The disc according to claim 1, wherein said load sharing means includes at least one pad member disposed and retained between said inner surfaces of said housing members and having cushioning and elastic properties for countering and thereby self centering against forces caused by relative movement of said housing member while under compressive forces are applied to said outer surfaces of said housing members.

3. The disc according to claim 2, wherein said pad members are made from a composition selected from the group consisting essentially of polymers and elastomers.

4. The disc according to claim 3, wherein said pad members are made from a composition selected from the group consisting essentially of silicone, polyurethane, urethane composites, plastics, polymers, and elastomers.

5. The disc according to claim 2, wherein said housing members includes seating means for seating said pad members between said inner surfaces.

6. The disc according to claim 5, wherein said seating means includes at least one pocket recessed into said inner surface of said housing members for seating a portion of said pad member therein.

7. The disc according to claim 6, further including adhering means for fixedly adhering said pad member within said pocket.

8. The disc according to claim 1, wherein the load sharing means are self-centering means for automatically centering self-adjusting bearing means within said housing members.

9. The disc according to claim 1, wherein said inner surfaces include at least one slot within each of said inner surfaces for seating said self-adjusting bearing means therein and allowing movement of said self-adjusting bearing means, further including bumper means for maintaining said bearing in alignment within said housing, said bumper means in sliding engagement with said slot.
